# EUROPEAN PATENT APPLICATION

(11) **EP 1 790 326 A1**
(43) Date of publication of application: **30.05.2007**
(21) Application number: 06255522.2
(22) Date of filing: 26.10.2006
(51) Int. Cl.: A61K 8/02, A61Q 19/10, A61K 8/73, A61K 8/86, A61K 8/81, A61K 8/365

(54) **Effervescent cleansing article**

(30) Priority: 27.10.2005 US 260606
(71) Applicant: Johnson and Johnson Consumer Companies, Inc., Skillman, NJ 08558 (US)
(72) Inventor: Luizzi, Joseph M., Newtown PA 18940 (US); Kundel, Nikhil K., Montclair NJ 07042 (US)
(74) Representative: James, Anthony Christopher W.P.

(57) **Abstract**

An article containing: a substrate having at least two surfaces; and
water-soluble elements on at least one surface of the substrate, wherein the water-soluble elements contain a material that effervesces upon contact with water is disclosed. The article is useful for cleansing and/or treating the skin and/or hair.

## Description

### BACKGROUND OF THE INVENTION

### 1. Field Of The Invention

The present invention relates to an effervescent cleansing article. The article contains water-soluble elements on at least one surface. The water-soluble elements contain a material that provides an effervescent reaction when wet. The water-soluble elements may provide cleansing and effervescing simultaneously. The substrate for the article may be a woven or knit fabric, a nonwoven, a laminate containing a fabric and a polymeric film, a flocked fabric, and combinations thereof.

### 2. Description Of The Prior Art

In recent years, many articles have been developed to aid in wiping various substrates. One example is the development of baby wipes, which are used to wipe the baby clean during diaper changes. Baby wipes typically are soft and are loaded with a cleanser and moisturizer.

Another type of wipe that has been developed is the hand wipe. Hand wipes are used to clean the hands when the use of a sink is inconvenient. These wipes typically are not as soft as baby wipes. Hand wipes frequently contain both cleansers and antibacterial agents.

United States Patent Nos. 5,538,732 and 6,001,380 relate to a method for applying a plurality of dermatological agents to the skin from a single dispensing and applicator sheet comprising a plurality of discrete areas comprising determatological agents which are simultaneously released from the sheet and applied to the afflicted skin area when the sheet is rubbed over wet skin.

United States Patent No. 6,878,380 relates to a wiping article which includes an effervescent cleanser composition held within a pouch formed from a first and second substrate sheet.

Despite the disclosure of the prior art, there remains a need for a wipe that cleans and simultaneously effervesces without the need for holding the effervescent composition within a pouch formed from a first and second substrate sheet. It has been discovered that when the effervescent composition is present on the surface of the wipe, in particular, in water-soluble elements present in discrete areas on the surface of the wipe, i.e., discrete water soluble elements, the effervescent effect is more readily apparent by the user. Because the effervescent material does not have to migrate through the pouch, the enhanced foaming/lathering provided by the effervescent material is more clearly visible to and felt more rapidly by the consumer.

### SUMMARY OF THE INVENTION

The present invention provides an article including a substrate having at least two surfaces; and water-soluble elements in discrete areas on at least one surface of the substrate, wherein the water-soluble elements contain a material that provides an effervescent reaction when contacted with water.

Also provided is a method for cleansing the skin, hair or nail involving wetting with water a cosmetic cleansing article including a substrate having at least two surfaces and water-soluble elements in discrete areas on at least one surface of the substrate, generating foam from the article and wiping skin surfaces with the wetted article. Other features and advantages of the present invention will be apparent from the detailed description of the invention and from the claims.

### DETAILED DESCRIPTION OF PREFERRED EMBODIMENTS

It is believed that one skilled in the art can, based upon the description herein, utilize the present invention to its fullest extent. The following specific embodiments are to be construed as merely illustrative, and not limitative of the remainder of the disclosure in any way whatsoever.

Unless defined otherwise, all technical and scientific terms used herein have the same meaning as commonly understood by one of ordinary skill in the art to which the invention belongs. Also, all publications, patent applications, patents and other references used herein are incorporated by reference. Whenever used, any percentage is weight by weight (w/w) unless otherwise indicated.

The articles of the present invention are preferably substantially dry. As used herein, "substantially dry" means that the article or composition contains less than about 10 percent, preferably less than about 5 percent, and more preferably less than about 1 percent by weight of unbound water, based on the total weight of the article or composition. In one embodiment, the article or composition contains less than less than about 10 percent, preferably less than about 5 percent, and more preferably less than about 1 percent by weight of total water (e.g., bound and unbound water), based on the total weight of the article or composition.

### Substrate

The article of the invention includes a substrate, preferably a water-insoluble substrate. By "water insoluble" is meant that the substrate, upon immersion in distilled water at 25°C, does not readily dissolve in or readily break apart. The water-insoluble substrate may, however, be disintegrated and/or dissolved slowly, e.g., over a period of several hours up to several days.

A wide variety of materials can be used as the substrate. Examples of suitable substrates include, but are not limited to, a woven fabric, a knit fabric, a nonwoven fabric, a laminate of a fabric and a polymeric film, such as a polyolefin film, a flocked fabric, hydroentangled substrates, air entangled substrates, natural sponges, synthetic sponges, polymeric netted meshes, and the like and combinations thereof. Methods of making woven and knit cloths are not a part of this invention and, being well known in the art, are not described in detail herein.

In one embodiment, the substrate contains a nonwoven material. By "nonwoven" is meant that the substrate, or a layer of the substrate, is comprised of fibers which are not woven into a fabric but rather are formed into a sheet, mat, or pad layer. The fibers can either be random (i.e., randomly aligned) or they can be carded (i.e. combed to be oriented in primarily one direction). Furthermore, the nonwoven substrate can be composed of a combination of layers of random and carded fibers.

Nonwoven substrates may be comprised of a variety of natural and/or synthetic materials. By "natural" is meant that the materials are derived from plants, animals, insects or byproducts of plants, animals, and insects. By "synthetic" is meant that the materials are obtained primarily from various man-made materials or from natural materials which have been further altered.

Nonlimiting examples of natural materials useful in the present invention are silk fibers, keratin fibers (such as wool fibers, camel hair fibers) and cellulosic fibers (such as wood pulp fibers, cotton fibers, hemp fibers, jute fibers, flax fibers, and mixtures thereof).

Examples of synthetic materials include, but are not limited to, those selected from the group containing of acetate fibers, acrylic fibers, cellulose ester fibers, modacrylic fibers, polyamide fibers, polyester fibers, polyolefin fibers, polyvinyl alcohol fibers, rayon fibers, polyurethane foam, and mixtures thereof.

Substrates made from natural and synthetic materials useful in the present invention can be obtained from a wide variety of commercial sources such as Freudenberg & Co. (Durham, NC USA), BBA Nonwovens (Nashville, TN USA), PGI Nonwovens (North Charleston, SC USA), Buckeye Technologies/Walkisoft (Memphis, TN USA), and Fort James Corporation(Deerfield, IL USA).

Methods of making nonwoven substrates are also well known in the art. Such methods include, but are not limited to, air-laying, water-laying, meltblowing, spinbonding, or carding processes. The resulting substrate, regardless of its method of production or composition, is then subjected to at least one of several types of bonding operations to anchor the individual fibers together to form a self-sustaining web. The nonwoven substrate can be prepared by a variety of processes including hydroentanglement, thermally bonding, and combinations of these processes. Moreover, the substrates can consist of a single layer or multiple layers. In addition, a multilayered substrate can include film layer(s) (e.g., apertured or non-apertured film layers) and other nonfibrous materials.

In one embodiment, the substrate is paper based. The materials for these substrates are made almost exclusively of cellulose-based fibres or filaments from plant cellular sources (pulp). These can be available from fresh wood-shavings or from recycled material (recycled paper).

If the substrate is to be used in a cleansing article (e.g., a facial or body wipe), high wet strength or firmness of the nonwoven material may be a desirable attribute. This can be achieved, for example, by the addition of binding materials, such as wet strength resins, or the material may be made of staple fibers, e.g. based on cotton, wool, linen and the like. Examples of wet strength resins include, but are not limited to, vinyl acetate-ethylene (VAE) and ethylene-vinyl chloride (EVCL) Airflex emulsions (Air Products, Lehigh, PA), Flexbond acrylic polymers (Air Products, Lehigh, PA), Rhoplex ST-954 acrylic binder (Rohm and Haas, Philadelphia, PA), and Ethylene-vinyl acetate (EVA) emulsion (DUR-O-SET® by National Starch Chemicals, Bridgewater, NJ). The amount of binding material in the substrate may range from about 5% to about 20%, by weight, of the substrate.

Nonwoven materials of increased strength can be obtained by using the so-called spunlace or hydro-entanglement technique. In this technique, the individual fibers are twisted together so that an acceptable strength or firmness is obtained without the need to use binding materials. The advantage of the latter technique is the excellent softness of the nonwoven material.

In one embodiment, the substrate contains a nonwoven material. By "nonwoven" is meant that the substrate, or a layer of the substrate, is comprised of fibers which are not woven into a fabric but rather are formed into a sheet, mat, or pad layer. The fibers can either be random (i.e., randomly aligned) or they can be carded (i.e. combed to be oriented in primarily one direction). Furthermore, the nonwoven substrate can be composed of a combination of layers of random and carded fibers.

Nonwoven substrates may be comprised of a variety of natural and/or synthetic materials. By "natural" is meant that the materials are derived from plants, animals, insects or byproducts of plants, animals, and insects. By "synthetic" is meant that the materials are obtained primarily from various man-made materials or from natural materials which have been further altered.

Nonlimiting examples of natural materials useful in the present invention are silk fibers, keratin fibers (such as wool fibers, camel hair fibers) and cellulosic fibers (such as wood pulp fibers, cotton fibers, hemp fibers, jute fibers, flax fibers, and mixtures thereof).

Examples of synthetic materials include, but are not limited to, those selected from the group containing of acetate fibers, acrylic fibers, cellulose ester fibers, modacrylic fibers, polyamide fibers, polyester fibers, polyolefin fibers, polyvinyl alcohol fibers, rayon fibers, polyurethane foam, and mixtures thereof.

Substrates made from natural and synthetic materials useful in the present invention can be obtained from a wide variety of commercial sources such as Freudenberg & Co. (Durham, NC USA), BBA Nonwovens (Nashville, TN USA), PGI Nonwovens (North Charleston, SC USA), Buckeye Technologies/Walkisoft (Memphis, TN USA), and Fort James Corporation(Deerfield, IL USA).

Methods of making nonwoven substrates are also well known in the art. Such methods include, but are not limited to, air-laying, water-laying, meltblowing, spinbonding, or carding processes. The resulting substrate, regardless of its method of production or composition, is then subjected to at least one of several types of bonding operations to anchor the individual fibers together to form a self-sustaining web.

One type of nonwoven cloth substrate utilized in the present invention is made by air- or water-laying processes in which the fibers or filaments are first cut to desired lengths from long strands, passed into a water or air stream, and then deposited onto a screen through which the fiber-laden air or water is passed. The deposited fibers or filaments are then adhesively bonded together, and otherwise treated as desired to form the woven, nonwoven, or cellulose cloth.

The nonwoven substrate can be prepared by a variety of processes including hydroentanglement, thermally bonding, and combinations of these processes. Moreover, the substrates can consist of a single layer or multiple layers. In addition, a multilayered substrate can include film layer(s) (e.g., apertured or non-apertured film layers) and other nonfibrous materials.

In one embodiment, the substrate is paper based. The materials for these substrates are made almost exclusively of cellulose-based fibres or filaments from plant cellular sources (pulp). These can be available from fresh wood-shavings or from recycled material (recycled paper).

If the substrate is to be used in a cleansing article (e.g., a facial or body wipe), high wet strength or firmness of the nonwoven material may be a desirable attribute. This can be achieved, for example, by the addition of binding materials, such as wet strength resins, or the material may be made of staple fibers, e.g. based on cotton, wool, linen and the like. Examples of wet strength resins include, but are not limited to, vinyl acetate-ethylene (VAE) and ethylene-vinyl chloride (EVCL) Airflex emulsions (Air Products, Lehigh, PA), Flexbond acrylic polymers (Air Products, Lehigh, PA), Rhoplex ST-954 acrylic binder (Rohm and Haas, Philadelphia, PA), and Ethylene-vinyl acetate (EVA) emulsion (DUR-O-SET® by National Starch Chemicals, Bridgewater, NJ). The amount of binding material in the substrate may range from about 5% to about 20%, by weight, of the substrate.

Nonwoven materials of increased strength can be obtained by using the so-called spunlace or hydro-entanglement technique. In this technique, the individual fibers are twisted together so that an acceptable strength or firmness is obtained without the need to use binding materials. The advantage of the latter technique is the excellent softness of the nonwoven material.

In another embodiment, the substrate utilized in the present invention may be a thermal bonded nonwoven cloth (whether or not resin-containing) which can be made of polyesters, polyamides, polyolefins, or other thermoplastic fibers which can be spun bonded, i.e., the fibers are spun out onto a flat surface and bonded (melted) together by heat or chemical reactions.

When nonwoven substrates are utilized, the nonwoven cloth substrates are generally adhesively bonded fibers or filamentous products having a web or carded fiber structure (when the fiber strength is suitable to allow carding) or comprising fibrous mats in which the fibers or filaments are distributed haphazardly or in random array (i.e., an array of fibers in a carded web where partial orientation of the fibers is frequently present, as well as a completely haphazard distributional orientation), or substantially aligned. The fibers or filaments can be natural (e.g., wool, silk, jute, hemp, cotton, linen, sisal, or ramie) or synthetic (e.g., rayon, cellulose ester, polyvinyl derivatives, polyolefins, such as polyethylene and polypropylene, polyamides, such as nylon 6, nylon 6,6, or polyesters, such as polyethylene terephthalate and polybutylene terephthalate), or combinations thereof. These nonwoven materials are generally described in the INDA "NONWOVEN FABRICS HANDBOOK", (1999), hereby incorporated by reference for nonwoven substrates and their methods of manufacture.

The basis weight of the substrate may vary, but generally ranges from about 20 grams per square meter to about 500 grams per square meter, for example from about 50 grams per square meter to about 150 grams per square meter.

Additives may also be added in order to increase the softness of the substrate. Examples of such additives include, but are not limited to, polyols such as glycerol, propylene glycol and polyethylene glycol, phthalate derivatives, citric esters, surfactants such as polyoxyethylene (20) sorbitan esters, and acetylated monoglycerides.

In one embodiment, the substrate is a woven substrate. Examples of woven substrates include, but are not limited to, woven cotton and polyester substrates. Examples of woven substrates include, but are not limited to, towels such a bath or hand towels and articles of clothing such as socks, mittens, gloves, and hats.

The substrate has at least two surfaces, generally a top surface and a bottom surface. The article of the invention is useful for cleansing the skin and simultaneously effervescing. In one embodiment, the article contains at least two water-soluble elements containing a material, that provides an effervescent reaction when contacted with water, in discrete areas on at least one surface of the substrate.

### Water-Soluble Elements

The water-soluble elements may be made of any suitable material. Suitable materials include, but are not limited to, polyethylene glycols ("PEGs") such as PEG-32 (CARBOWAX 1450) and PEG-765 (CARBOWAX 3350) from Union Carbide (Union Carbide, Midland, MI); polyethylene oxides such as PEG-2M (POLYOX WSRN-10) and PEG-5M (POLYOX WSRN-80) from Amerchol (Edison, NJ); polyvinyl alcohols such as PVAXX resins C-20 and W-20 (Mitsui Plastics, White Plains, NY USA); polysaccharides, such as alginic acid and its alkali metal and ammonium salts; alkylcelluloses, such as methylcellulose and ethylcellulose; cellulose ethers such as hydroxypropylcellulose, hydroxymethylcellulose, hydroxyethylcellulose, and hydroxybutylcellulose; hydroxyethylmethylcellulose; hydroxypropylmethylcellulose; carboxyalkylcellulosics, such as carboxymethylcellulose; polyvinylpyrrolidone; copolymers of vinyl pyrrolidone and vinyl acetate such as PLASDONE S-630 (ISP, Wayne, NJ, USA); and mixtures thereof. Polyethylene glycols, polyethylene oxides, and mixtures thereof are preferred. Colorants or pigments may be combined with the water-soluble materials.

### Effervescing Material

The article of the invention includes at least one material that provides an effervescent reaction when wet. Effervescent compositions are well known in the art. Generally, an acid is utilized to neutralize a carbonate salt, resulting in evolution of carbon dioxide gas, which generates the effervescence. The reaction takes place in the presence of water. In order to prevent pre-mature reaction of the acid and the carbonate salt, anhydrous materials are preferred. It is also preferred to keep the materials dry until ready for use.

Suitable carbonate salts include, but are not limited to, sodium carbonate, potassium carbonate, magnesium carbonate, sodium bicarbonate, potassium bicarbonate, magnesium bicarbonate, and blends thereof. A blend of 50 percent by weight sodium carbonate and 50 percent by weight sodium bicarbonate is preferred.

Suitable acids include, but are not limited to, citric acid, fumaric acid, adipic acid, malic acid, and combinations thereof. The ratio of acid to carbonate will affect the rate of effervescence. The ratio of acid to carbonate generally may range from about 1 to 10 to about 10 to 1. An acid to carbonate ratio of 1 to 1 is preferred.

To initiate the effervescent reaction, the article must be wet with water, e.g., water must come in contact with the carbonate salt and the acid. The water may be added prior to application (e.g., wetting the article with tap water), during application (e.g., applying the article to water on the skin, hair, or teeth), or after application (e.g., skin perspiration being absorbed into the composition or article).

### Configurations of the Effervescent Article

The water-soluble material and the effervescent material are typically mixed prior to being applied to the substrate. The water-soluble elements comprising the effervescent material may be applied onto the substrate by any means known in the art, such as control coating, control fiberization, pattern coating, gravure coating, rotary screen printing, and spray coating. Equipment for coating the substrates is commercially available. One example is the DYNAFIBER, available through Nordson Company. Another example is the ITW, available through Omega Company. When applying water-soluble elements through a melt process, the time it takes to cool the applied coating affects the height of the water-soluble elements. If the coating is not cooled quickly enough, the coating may penetrate the substrate to the extent that no raised element is formed. If raised elements are desired, to overcome this problem, an air knife that utilizes air, which may be chilled, may be utilized to quickly cool the applied coating and prevent tailing. The angle of contact between the air and the applied coating may also affect the height of the raised elements. The air typically contacts the coating at an angle of from about 10° to about 80°.

The water-soluble elements may be of any shape including, but not limited to, lines, waves, interconnected patterns, circular dots, hexagons, hearts, diamonds, rectangles, stars, triangles and the like. In one embodiment, the wipes are used to cleanse or treat sensitive body surfaces, such as, the face. In such an embodiment, the shape of the water-soluble elements must be such as not to provide irritation to the skin. For example, shapes such as a tail, scoop or prong should be avoided. The density, height, and diameter of the water-soluble elements may vary depending upon the desired feel of the wipe and the desired benefit to be achieved. Generally, the water-soluble elements may occupy anywhere from about 1 percent to about 99 percent, for example from about 10 percent to about 75 percent, or from about 20 percent to about 50 percent of the surface of at least one side of the substrate. Generally, the diameter of the water-soluble elements may range from about 0.5 mm to about 3 mm, for example about 1 mm to about 2 mm.

The articles of the present invention may provide a skin care benefit or a hair care benefit. As used herein, skin care benefit and hair care benefit means cleansing or treating the skin or hair. The articles of the invention are preferably combined with cleansers, skin care actives, moisturizers, and the like.

### Surfactants

In one embodiment, the article or composition further contains one or more surfactants. In one embodiment, the article or composition contains a lathering surfactant. What is meant by a lathering surfactant is a surfactant that generates lather when combined with water and mechanically agitated. In one embodiment, the lathering surfactant has an initial foam height reading of at least about 20 mm, such as at least about 50 mm, in the Standard Test Method for Foaming Properties of Surface-Active Agents D1173-53 Set forth in the ASTM Annual Book of ASTM Standards 1001 Section 15 Volume 15.04 (using a concentration of 5 grams per liter, temperature of 49°C, and water hardness of 8 grains per gallon). Examples of lathering surfactants include, but are not limited to, anionic, nonionic, cationic, and amphoteric lathering surfactants.

Nonlimiting examples of anionic lathering surfactants include those selected from the group consisting of sarcosinates, sulfates, isethionates, taurates, phosphates, lactylates, and glutamates. Specific examples include, but are not limited to, sodium lauryl sulfate, ammonium lauryl sulfate, ammonium laureth sulfate, sodium laureth sulfate, sodium trideceth sulfate, ammonium cetyl sulfate, sodium cetyl sulfate, ammonium cocoyl isethionate, sodium lauroyl isethionate, sodium lauroyl lactylate, triethanolamine lauroyl lactylate, sodium caproyl lactylate, sodium lauroyl sarcosinate, sodium myristoyl sarcosinate, sodium cocoyl sarcosinate, sodium lauroyl methyl taurate, sodium cocoyl methyl taurate, sodium lauroyl glutamate, sodium myristoyl glutamate, and sodium cocoyl glutamate and mixtures thereof

Nonlimiting examples of nonionic lathering surfactants include alkyl glucosides, alkyl polyglucosides, polyhydroxy fatty acid amides, alkoxylated fatty acid esters, lathering sucrose esters, amine oxides, and mixtures thereof. Specific examples include, but are not limited to, nonionic surfactants such as C8-C14 glucose amides, C8- C14 alkyl polyglucosides, sucrose cocoate, sucrose laurate, lauramine oxide, cocoamine oxide, and mixtures thereof.

Nonlimiting examples of amphoteric lathering surfactants (which also includes zwitterionic lathering surfactants) are betaines, sultaines, hydroxysultaines, alkyliminoacetates, iminodialkanoates, aminoalkanoates, and mixtures thereof.

Nonlimiting examples of amphoteric surfactants of the present invention include disodium lauroamphodiacetate, sodium lauroamphoacetate, cetyl dimethyl betaine, cocoamidopropyl betaine, cocoamidopropyl hydroxy sultaine, and mixtures thereof.

### Bulking Agents

In one embodiment, the composition or article further contains a bulking agent. Examples of bulking agents include, but are not limited to, talc, clays such as aluminum silicates, cellulose pulps, silicas, and starches such as corn starch. Other bulking agents are disclosed on pages 1625-26 of the INCI Handbook. The amount of bulking agent in the composition may range from about 5% to about 99.5%, by weight, of the composition.

### Cosmetically Active Agents

In one embodiment, the composition or article further contains a cosmetically active agent(s). What is meant by a "cosmetically active agent" is a compound (e.g., a synthetic compound or a compound isolated from a natural source) that has a cosmetic or therapeutic effect on the skin, mucosa, teeth, hair, or nails, including, but not limited to, lightening agents, darkening agents such as self-tanning agents, anti-acne agents, shine control agents, anti-microbial agents, anti-inflammatory agents, anti-mycotic agents, anti-parasite agents, external analgesics, sunscreens, photoprotectors, antioxidants, keratolytic agents, detergents/surfactants, moisturizers, nutrients, vitamins, energy enhancers, anti-perspiration agents, astringents, deodorants, hair removers, firming agents, anti-callous agents, and agents for hair, nail, mucosa, teeth, and/or skin conditioning.

In one embodiment, the agent is selected from, but not limited to, hydroxy acids, benzoyl peroxide, sulfur resorcinol, ascorbic acid, D-panthenol, hydroquinone, octyl methoxycinnimate, titanium dioxide, octyl salicylate, homosalate, avobenzone, polyphenolics, carotenoids, free radical scavengers, spin traps, retinoids such as retinol and retinyl palmitate, ceramides, polyunsaturated fatty acids, essential fatty acids, enzymes, enzyme inhibitors, minerals, hormones such as estrogens, steroids such as hydrocortisone, 2-dimethylaminoethanol, copper salts such as copper chloride, peptides containing copper such as Cu:Gly-His-Lys and coenzyme Q10, lipoic acid, amino acids such a proline and tyrosine, vitamins, lactobionic acid, acetyl-coenzyme A, niacin, riboflavin, thiamin, ribose, electron transporters such as NADH and FADH2, and other botanical extracts such as aloe vera and legumes such as soy beans, and derivatives and mixtures thereof. The cosmetically active agent will typically be present in the composition or article of the invention in an amount of from about 0.001 % to about 20% by weight of the composition, e.g., about 0.01 % to about 10% such as about 0.1 % to about 5%.

Examples of vitamins include, but are not limited to, vitamin A, a vitamin B such as vitamin B3, vitamin B5, and vitamin B12, vitamin C, vitamin K, and vitamin E and derivatives thereof.

Examples of hydroxy acids include, but are not limited, to glycolic acid, lactic acid, malic acid, salicylic acid, citric acid, and tartaric acid. See, e.g., European Patent Application No. 273,202.

Examples of antioxidants include, but are not limited to, water-soluble antioxidants such as sulfhydryl compounds and their derivatives (e.g., sodium metabisulfite and N-acetyl-cysteine), lipoic acid and dihydrolipoic acid, resveratrol, lactoferrin, and ascorbic acid and ascorbic acid derivatives (e.g., ascorbyl palmitate and ascorbyl polypeptide). Oil-soluble antioxidants suitable for use in the compositions of this invention include, but are not limited to, butylated hydroxytoluene, retinoids (e.g., retinol and retinyl palmitate), tocopherols (e.g., tocopherol acetate), tocotrienols, and ubiquinone. Natural extracts containing antioxidants suitable for use in the compositions of this invention, include, but not limited to, extracts containing flavonoids and isoflavonoids and their derivatives (e.g., genistein and diadzein), extracts containing resveratrol and the like. Examples of such natural extracts include grape seed, green tea, pine bark, and propolis. Other examples of antioxidants may be found on pages 1612-13 of the INCI Handbook.

### Anti-acne Agent

In one embodiment, the article or composition of the present invention includes an anti-acne agent(s). What is meant by an "anti-acne agent" is a drug product effective is the treatment of acne. Examples of anti-acne agents include, but are not limited to, azelaic acid, clindamycin, adapalene, erythromycin, sodium sulfacetamide, retinoic acid, benzoyl peroxide, sulfur, and salicylic acid.

In one embodiment, the article or composition includes about 0.1 to about 50 percent, by weight, of the at least one anti-acne agents, e.g., about 0.5 to about 30 percent, by weight, such as about 0.5 to about 15 percent, by weight, of the at least one anti-acne agent.

In one embodiment, the composition further contains a natural extract to enhance the anti-acne efficacy of the anti-acne agent. Examples of such extracts include, but are not limited to, angelica archangelica root extract, dandelion extract, turmeric extract, and melia azadirachta leaf extract.

### pH Adjusters

As the reducing agents and oxidizing agents often exhibit alkaline pH values in the range of from about 9 to about 11 when dissolved in water, the resulting solution created after the exothermic reaction is complete would leave an alkaline pH to the surface being exposed to the agents, which for example could be damaging (e.g., to the stratum corneum). Thus, in one embodiment, the substrate contains an acid or buffering agent (e.g., citric acid) to maintain the pH of the solution created by wetting the composition or article to be in the range of from about 6 to about 8 (e.g., from about 6.5 to about 7.5).

### Other Materials

Various other materials may also be present in the compositions and articles useful in the subject invention. These include humectants, emollients, chelating agents (e.g., EDTA), preservatives (e.g., parabens) and medicinal agents. Examples of such are listed in pp. 1654-62 and 1626, of the INCI Handbook. In addition, the topical compositions useful herein can contain conventional cosmetic adjuvants, such as dyes, opacifiers (e.g., titanium dioxide and zinc oxide), pigments, and fragrances.

Typical medicinal agents useful in the present invention include but are not limited to oatmeal, bicarbonate of soda, colloidal oatmeal, oilated colloidal bath treatment, surfactant based colloidal oatmeal cleanser, other cleanser systems, soy powder, herbal medicines and combinations thereof.

Skin care or hair care compositions may be loaded onto the wipes prior to coating the wipe with the water-soluble elements by dipping the wipes in the skin care or hair care composition, spraying the skin care or hair care composition onto the wipes, and other means known in the art. The wet wipe may be dried through the use of heating equipment or vacuum driers to provide dry wipes. Alternatively, a hair care or skin care formulation may be applied in the form of a concentrate to the substrate to provide dry textured articles. In an alternative embodiment, the skin care or hair care composition may be blended with the water-soluble material and the effervescent material, then applied to the substrate, cooled and dried. The articles or wipes may be sold dry, then the consumer wets the wipe with water when ready for use.

Several examples are set forth below to further illustrate the nature of the invention and the manner of carrying it out. However, the invention should not be considered as being limited to the details thereof.

### Example 1:

A cleansing and effervescent article was prepared utilizing JACOB HOLMES 100% polyester fabric having a basis weight of 70 grams per square meter as the substrate. The following materials were combined to create a "stock blend":
91.5% CARBOWAX 1450, 6.5% POLYOX 1105, 1.99% calcium carbonate, and 0.01% FD&C Blue #1. The CARBOWAX 1450 was heated to 70° - 80° C and stirred. The FD & C Blue #1 was added to the heated CARBOWAX 1450 and stirred. The POLYOX 1105 was slowly added to the mixture while stirring. Finally, the calcium carbonate was added to the mixture and stirred.

Molten stock blend (270 grams) was placed into a vessel equipped with stirring. To the stock blend was added 7.5 grams of anhydrous sodium carbonate, 7.5 grams of sodium bicarbonate, and 15 grams of citric acid. The mixture was stirred to make the water-soluble effervescent coating material. The water-soluble effervescent coating material was screen coated onto the polyester fabric at an add-on weight of 40 grams per square meter. The coating material was applied in a dot pattern. The diameter of the dots was 0.06 inches. The spacing between the dots was 0.125 inches. There were 64 dots per square inch. The coated substrate was allowed to cool.

The coated wipe was wetted with water from a sink and used to cleanse the hands and arms of a subject. The subject noticed effervescence immediately upon wetting the wipe and throughout the cleansing.

### Example 2:

A second cleansing and effervescent article was prepared utilizing JACOB HOLMES 100% polyester fabric having a basis weight of 70 grams per square meter as the substrate. Molten stock blend (150 grams as prepared in Example 1) was placed into a vessel equipped with stirring. To the stock blend was added 37.5 grams of anhydrous sodium carbonate, 37.5 grams of sodium bicarbonate, and 75 grams of citric acid. The mixture was stirred to make the water-soluble effervescent coating material. The water-soluble effervescent coating material was screen coated onto the polyester fabric at an add-on weight of 40 grams per square meter. The coating material was applied in a dot pattern. The diameter of the dots was 0.06 inches. The spacing between the dots was 0.125 inches. There were 64 dots per square inch. The coated substrate was allowed to cool.

The coated wipe was wetted with water from a sink and used to cleanse the hands and arms of a subject. The subject noticed effervescence immediately upon wetting the wipe and throughout the cleansing. The amount of effervescence was significantly greater than that with the sample of Example 1.

## Claims

1. An article comprising: a substrate having at least two surfaces; and water-soluble elements in discrete areas on at least one surface of the substrate, wherein the water-soluble elements contain a material that provides an effervescent reaction when contacted with water.

2. The article according to claim 1 wherein the substrate is selected from the group consisting of a woven fabric, a knit fabric, a nonwoven fabric, a laminate of a fabric and a polymeric film, a flocked fabric, and combinations thereof.

3. The article according to claim 1 wherein the water-soluble elements comprise a material selected from the group consisting of polyethylene glycols; polyethylene oxides; polyvinyl alcohols; polysaccharides; alkylcelluloses; cellulose ethers; hydroxyethylmethylcellulose; hydroxypropylmethylcellulose; carboxyalkylcellulosics; polyvinylpyrrolidone; copolymers of vinyl pyrrolidone and vinyl acetate; and mixtures thereof.

4. The article according to claim 3 wherein the water-soluble elements comprise a material selected from the group consisting of polyethylene glycols, polyethylene oxides, and mixtures thereof.

5. The article according to claim 1 wherein the material that provides an effervescent reaction when contacted with water is a combination of an acid and a carbonate salt.

6. The article according to claim 1 wherein the water-soluble elements are in a shape selected from circular dots, hexagons, hearts, diamonds, rectangles, stars, and triangles.

7. The article according to claim 2 wherein the substrate has a basis weight ranging from about 20 grams per meter to about 500 grams per square meter.

8. The article according to claim 6 wherein the water-soluble elements cover from about 10 percent to about 90 percent of the surface of at least one side of the substrate.

9. The article according to claim 6 wherein the water-soluble elements have a diameter ranging from about 0.5 mm to about 3 mm.

10. The article according to claim 1 further comprising a lathering surfactant.

11. A method of providing a cleansing and effervescing wipe comprising: providing a substrate selected from the group consisting of a woven fabric, a knit fabric, a nonwoven fabric, a laminate of a fabric and a polymeric film, a flocked fabric, and combinations thereof; and pattern coating water-soluble elements onto the substrate, wherein the water-soluble elements contain a material that provides an effervescent reaction when contacted with water.

12. The method according to claim 11 wherein the water-soluble elements further comprise a lathering surfactant.

13. A method for cleansing the skin or hair comprising topically applying the article of claim 1 to said skin or hair.

14. A method according to claim 13, wherein said skin is the face.
